# EUROPEAN PATENT APPLICATION

(11) **EP 0 771 871 A2**
(43) Date of publication of application: **07.05.1997**
(21) Application number: 97101044.2
(22) Date of filing: 25.04.1989
(51) Int. Cl.: C12N 15/00, C12P 21/02

(54) **Expression of human serum albumin in methylotrophic yeasts**

(30) Priority: 25.04.1988 US 19880186420
(62) Divisional of application: 89107459.3
(71) Applicant: Research Corporation Technologies, Inc, Tucson, Arizona 85711-3335 (US)
(72) Inventor: Marashi, Farhad, Bartlesville, OK 74006 (US); Wong, Ricky Ngok-Shun, Plano, TX 75023 (US); Fuke, Motohiro, Tulsa, OK 74137 (US); Davis, Frances Marie, Houston, TX 77025 (US); McCombie, William Richard, Bartlesville, OK 74006 (US); Vineyard, Karen Marie, Bartlesville, OK 74006 (US); Barr, Robert Dean, Bartlesville, OK 74003 (US); Parker, Kathryn Ann, Bartlesville, OK 74003 (US); Sreekrishna, Kotikanyadan, Bartlesville, OK 74006 (US)
(74) Representative: Dost, Wolfgang, Dr.rer.nat., Dipl.-Chem., et al

(57) **Abstract**

A process for the production of HSA. Also disclosed are DNA molecules and methylotrophic yeasts transformed with the molecules. Additionally disclosed is a novel HSA nucleotide sequence.

## Description

### Field of the Invention

This invention relates to the field of recombinant DNA biotechnology. In one aspect, this invention relates to a process for the expression of human serum albumin (HSA) in methylotrophic yeasts. In another aspect the present invention relates to novel DNA molecules and novel yeast strains transformed therewith.

### Background

Human serum albumin is the most abundant plasma protein of adults. The concentration of albumin is 40 mg/ml, or 160g of albumin circulating throughout the human body for a 70 Kg adult male. This protein maintains osmotic pressure and functions in the binding and transport of copper, nickel, calcium (weakly, at 2-3 binding sites) bilirubin and protoporphyrin, long chain fatty acids, prostaglandins, steroid hormones (weak binding with these hormones promotes their transfer across the membranes), thyroxine, triiodothyronine, cystine, and glutathione. According to Peters et al, over 10,000 kilograms of purified albumin are administered annually in the United States alone to patients with circulatory failure or with albumin depletion.

Currently the only commercial source of HSA is from fractionalized blood. Considering the possible dangers of blood borne contaminants and pathogens, it would be a considerable contribution to the commercial production of HSA to develop alternate methods of producing HSA. With the advent of recombinant DNA biotechnology, it is now possible to produce HSA by alternate methods.

Unfortunately, although HSA has been produced in E.coli cells there are significant disadvantages to producing HSA in this host. For example, E. coli produces endotoxins which must be removed by expensive purification steps.

Thus it would be a significant contribution to the art to develop a process for the production of HSA.

Therefore, it is an object of this invention to provide a process for the enhanced production of HSA.

Yet another object of this invention is to provide novel vectors containing DNA sequences which code for HSA.

A further object of this invention is to provide novel methylotrophic yeasts transformed with a vector or vectors capable of enhanced production of HSA.

A still further object of this invention is a novel nucleotide sequence coding for HSA.

### Summary of the Invention

In accordance with the present invention, we have discovered a process for the production of HSA comprising transforming a methylotrophic yeast with at least one vector having a compatible expression cassette containing a structural gene for HSA thereof, and culturing the resultant transformants under conditions suitable to obtain the production of HSA and a novel nucleotide sequence coding for HSA.

### Detailed Description of the Figures

Figure 1 provides a representation of plasmid pA0804 which contains a linear integrative site-specific vector in the fragment clockwise from BgIII to BGIII. The structural gene may be inserted in the unique EcoRI site of this plasmid. This plasmid may be recovered from the plasmid DNA of NRRL B-18114 by an EcoRI digest and gel electrophoresis to recover a linear ∼7.4 Kb EcoRI fragment corresponding to Figure 1.
Figure 2(a) provides a linear map of pA0804.
Figure 2(b) provides linear map of pA0807, a derivative of pA0804 containing an fl-ori of approximately 458 base pairs.
Figure 2(c) provides a linear map of pA0807N which has NotI sites inserted in place of the BgIII sites of pA0807.
Figure 2(d) provides linear map of pHSA113 which is a linear map of pA0807N with an HSA gene inserted in the unique EcoRI site.
Figure 3 provides a representation of pA0807N in circular form.
Figure 4 provides a representation of plasmid pTHFKΔ an autonomous yeast plasmid DNA of NRRL B-18115 by an EcoRI digestion gel electrophoresis and recovering the 6.2 KG EcoRI fragment.

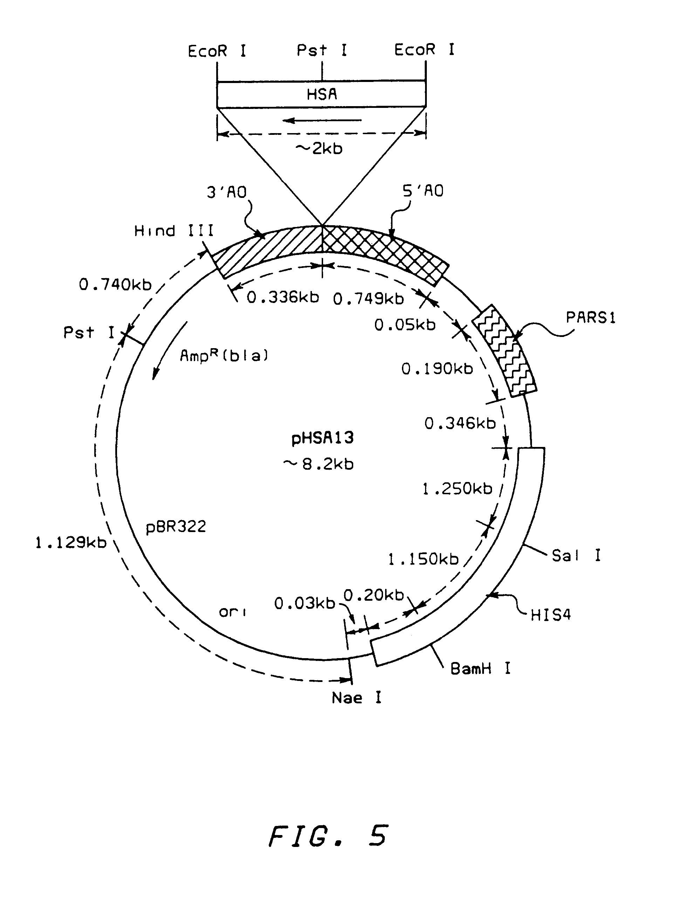

Figure 5 provides a representation of pHSA13, which is a derivative of pTHFKΔ containing a HSA gene inserted in the unique EcoRI site of pTHFKΔ.

### Detailed Description

The HSA structural genes have been sequenced by Lawn et al. Nuc. Acids. Res. 9:6105 (1981), Dugaiczyk et al. Proc. Natl. Acad. Sci. USA 79:71 (1982).

This gene may be obtained by reisolation of the gene by the technique of Lawn et al., Dugaiczyk et al. or as described in Example I or synthesized in vitro by a custom gene manufacturer such as British Biotechnology, Ltd. One possible method of obtaining the HSA gene would be to screen a liver cDNA library with oligonucleotide probes and optionally with immunoscreening positive plaques for the HSA structural gene. Performing this type of isolation scheme, an HSA gene was isolated having the nucleotide sequence shown in Table 1.

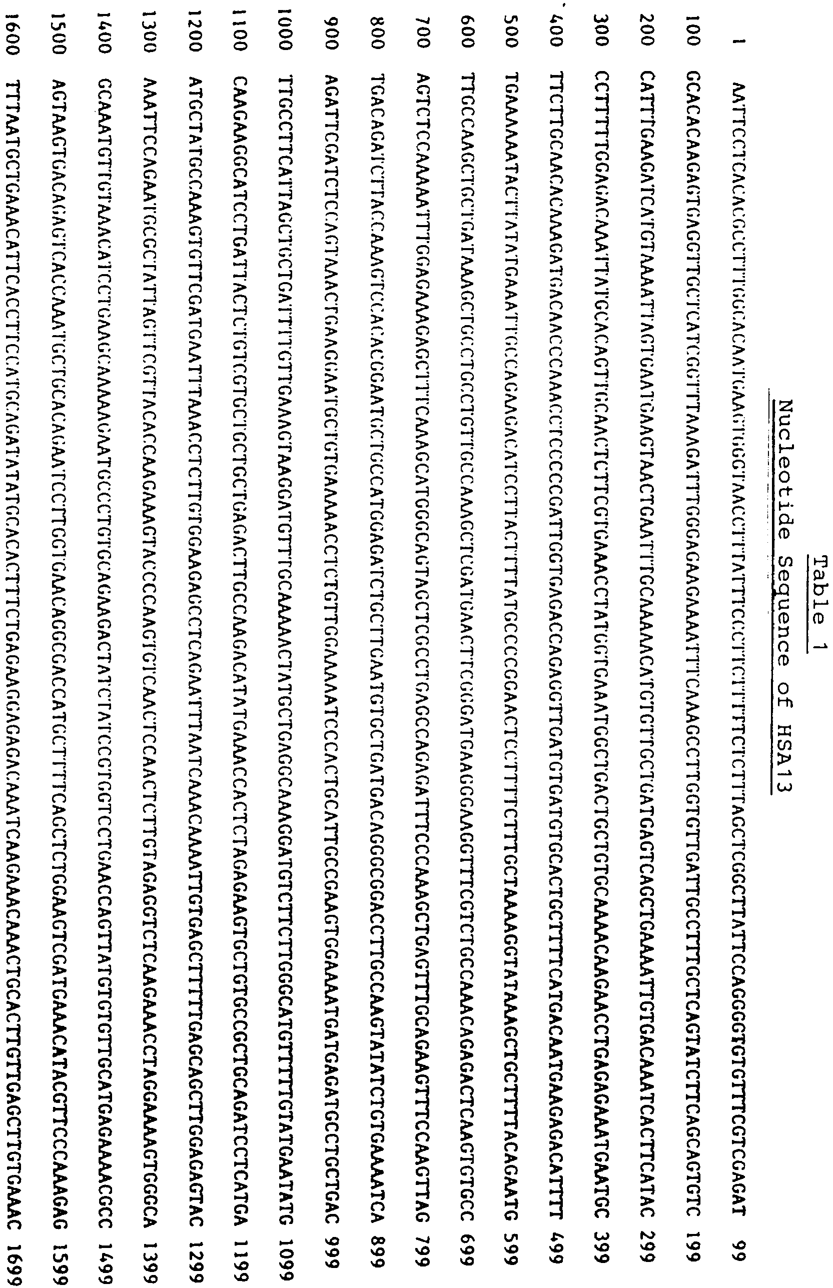

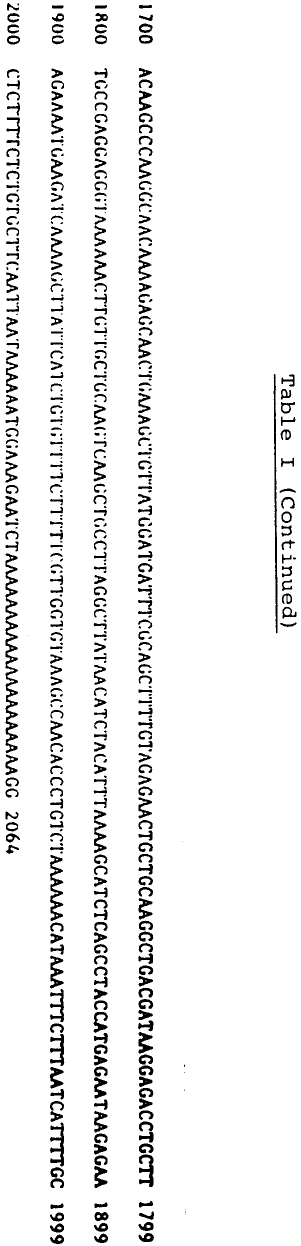

The nucleotide sequence provided in Table 1 was obtained by sequencing the isolated HSA, DNA, which may be performed by any suitable technique such as the dideoxynucleotide chain termination method of Sanger et al., PNAS 74, 5463 (1977), or by subcloning using M13 derivatives, sequencing as is described by Sanger et al., J. Mol. Biol. 142, 1617 (1980).

This sequence is a novel nucleotide sequence compared to the sequences published by Lawn et al. and Dugaiczyk et al. A comparison of the nucleotide sequences is provided in Table 2.

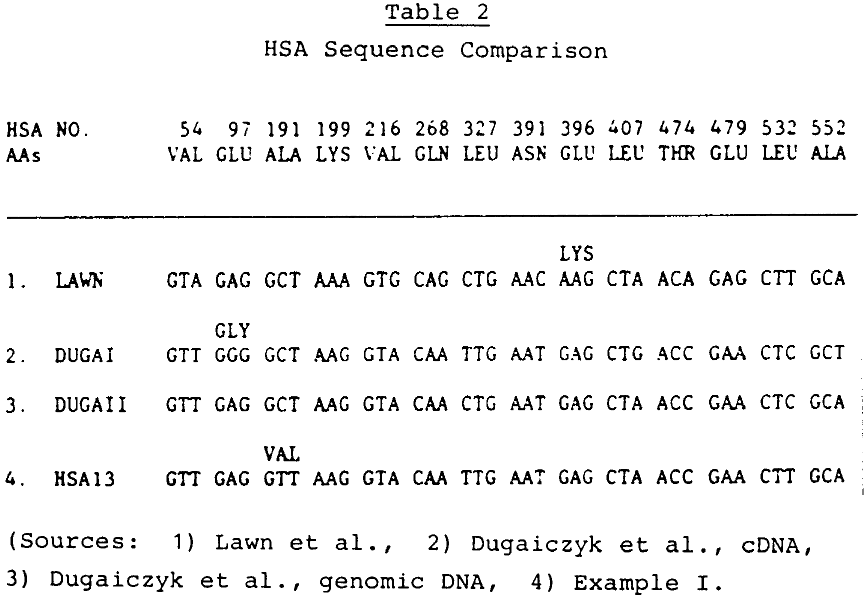

Once the structural gene for HSA is recovered, it may first be necessary to insert the structural gene into a vector and a compatible host cell line to propagate the gene, or to further tailor the gene and the like.

Culturing the host may be accomplished by any suitable means. General techniques for culturing hosts are already known in the art and any adaptation of these methods to the specific requirements of the strains used herein is well within the abilities of those skilled in the art.

Recovery of plasmid DNA from hosts can be accomplished by several techniques due to its compact size and closed circular superhelical form. For example, following the harvest host cells may be pelleted by centrifugation and then resuspended and lysed. The lysate should be centrifuged to remove cell debris and the supernatant containing DNA retained. A phenol extraction can then be performed to remove most other contaminants from the DNA. The phenol-extracted DNA may then be further treated using a density gradient centrifugation or a gel filtration technique to separate the plasmid DNA from the bacterial DNA. The techniques for achieving the separation alluded to above are well known in the art and numerous methods for performing these techniques are known.

Nuclease digestion of the plasmids may be accomplished by choosing appropriate endonucleases which will cut the selected plasmid in such a way as to facilitate the recovery of the HSA structural gene. The endonucleases used will depend on the plasmid from which the HSA gene is to be excised.

Gel electrophoresis of DNA may be accomplished using numerous techniques. See P.G. Sealy and E.M. Southern, Gel Electrophoresis of Nucleic Acids - A Practical Approach (D. Rickwood and B.D. Hames, eds.) p. 39 (1982). Elution may also be accomplished using numerous techniques appropriate for the gel involved, such as electroelution, diffusion, gel dissolution (agarose gels) or physical extrusion (agarose gels). It is additionally recognized the elution may not be necessary with some gels such as high-quality, low melting temperature agarose.

Once the fragment containing the HSA structural gene or fragments thereof is isolated, additional manipulations may be required before it is inserted in the vector. These manipulations may include, but are not limited to the addition of linkers or blunt-ending the fragment.

Following the isolation of the HSA structural gene, the gene is inserted into a suitable methylotrophic yeast vector such as a plasmid or linear site-specific integrative vector. Preferably vectors for the practice of this invention are those compatible with the Pichia genus and most preferably Pichia pastoris.

Plasmids have long been one of the basic elements employed in recombinant DNA technology. Plasmids are circular extrachromosomal double-stranded DNA found in microorganisms. Plasmids have been found to occur in single or multiple copies per cell. Included in plasmid DNA is the information required for plasmid reproduction, i.e. an autonomous replication sequence such as those disclosed by Cregg in European Application 0180899 published May 14, 1986. One or more means of phenotypically selecting the plasmid in transformed cells may also be included in the information encoded in the plasmid. Phenotypic or selection markers, such as antibiotic resistance genes or genes which complement defects in the host biochemical pathways, permit clones of the host cells which have been transformed to be recognized, selected, and maintained.

To express the HSA structural gene in methylotrophic yeasts, the gene must be operably linked to a 5' regulatory region and 3' termination sequence, which forms the expression cassette which will be inserted into the host via a vector.

The following terms are defined herein for the purpose of clarification.

Operably linked--refers to a juxtaposition wherein the components are configured so as the perform their function.

Regulatory region--DNA sequences which respond to various stimuli and affect the rate of mRNA transcription.

3' Termination sequence--sequences 3' to the stop codon which function to stabilize the mRNA such as sequences which elicit polyadenylation.

"Pichia compatible" refers to DNA sequences which will perform their normal function in Pichia such as regulatory regions and 3' termination sequences derived from Pichia.

Integrative vectors, such as the linear site-specific integrative vector of Cregg, as described in European Application Serial Number 86114700.7. Such vectors comprise a serially arranged sequence of at least 1) a first insertable DNA fragment; 2) a selectable marker gene; and 3) a second insertable DNA fragment may also be used for the practice of this invention.

The first and second insertable DNA fragments are each at least about 200 nucleotides in length and have nucleotide sequences which are homologous to portions of the genomic DNA of the species to be transformed. The various components of the integrative vector are serially arranged forming a linear fragment of DNA such that the expression cassette and the selectable marker gene are positioned between the 3' end of the first insertable DNA fragment and the 5' end of the second insertable DNA fragment. The first and second insertable DNA fragments are oriented with respect to one another in the serially arranged linear fragment as they are so oriented in the parent genome.

Nucleotide sequences useful as the first and second insertable DNA fragments are nucleotide sequences which are homologous with separate portions of the native genomic site at which genomic modification is to occur. Thus, for example, if genomic modification is to occur at the locus of the alcohol oxidase gene, the first and second insertable DNA fragments employed are homologous to separate portions of the alcohol oxidase gene locus. For genomic modification in accordance with the present invention, the two insertable DNA fragments must be oriented with respect to one another in the linear fragment in the same relative orientation as they exist in the parent genome. Examples of nucleotide sequences which could be used as first and second insertable DNA fragments are nucleotide sequences selected from the group consisting of the alcohol oxidase (AOXI) gene, dihydroxyacetone synthase (DHAS) gene, p40 gene and HIS4 gene. The AOXI gene,DHAS gene, p40 gene and HIS4 gene are contained in copending application Serial Number 780,102 incorporated herein by reference.

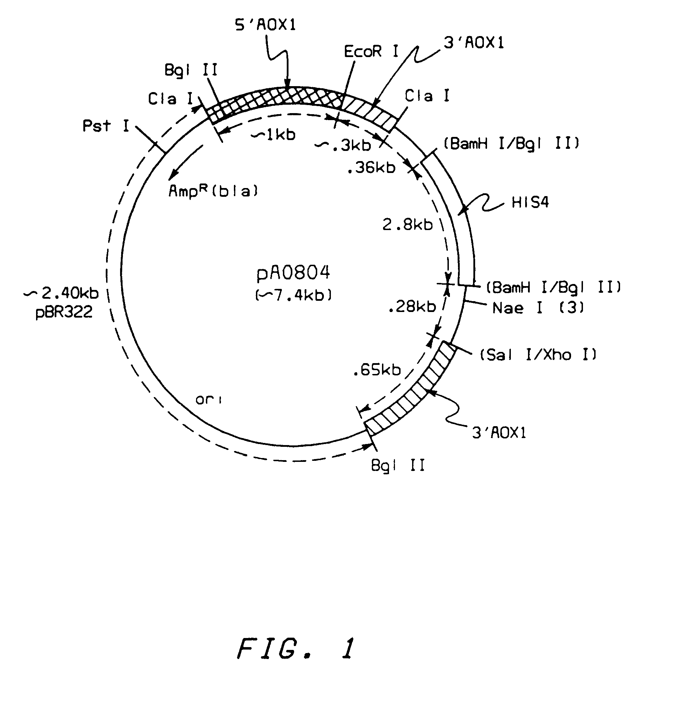

The first insertable DNA fragment may contain an operable regulatory region which may comprise the regulatory region utilized in the expression cassette. The use of the first insertable DNA fragment as the regulatory region for an expression cassette is a preferred embodiment of this invention. Figure 1 provides a diagram of a vector utilizing the first insertable DNA fragment as a regulatory region for a cassette.

Optionally as shown in Figure 1 an insertion site or sites and a 3' termination sequence may be placed immediately 3' to the first insertable DNA fragment. This conformation of the linear site-specific integrative vector has the additional advantage of providing a ready site for insertion of a structural gene without necessitating the addition of a compatible 3' termination sequence.

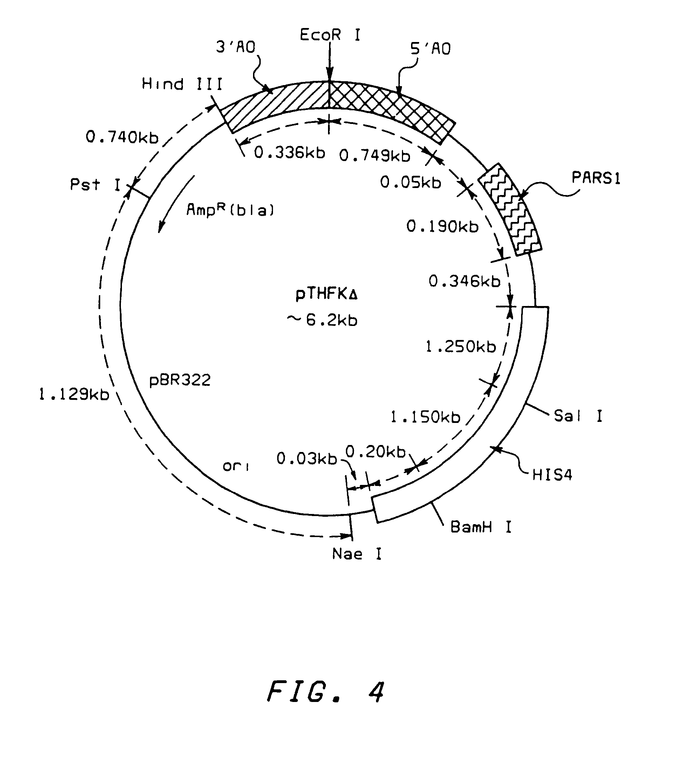

Additionally the expression vectors used in the practice of this invention could contain a polylinker site to facilitate the insertion of structural genes or cassettes or the like, between the first insertable DNA fragment and the second insertable DNA fragment, or the regulatory region and termination sequence as shown in Figure 4.

It is also necessary to include at least one selectable marker gene in the DNA used to transform the host strain. This facilitates selection and isolation of those organisms which have incorporated the transforming DNA. The marker gene confers a phenotypic trait to the transformed organism which the host did not have, e.g., restoration of the ability to produce a specific amino acid where the untransformed host strain has e defect in the specific amino acid biosynthetic pathway or resistance to antibiotics and the like.

Exemplary selectable marker genes may be selected from the group consisting of the HIS4 gene and the ARG4 gene from Pichia pastoris and Saccharomyces cerevisiae, the invertase gene (SUC2) from Saccharomyces cerevisiae, or the G418^{R}/kanamycin resistance gene from the E. coli transposable elements Tn601 or Tn903.

Those of skill in the art recognize that additional DNA sequences can also be incorporated into the vectors employed in the practice of the present invention, such as for example, bacterial plasmid DNA, bacteriophage DNA, and the like. Such sequences enable the amplification and maintenance of these vectors in bacterial hosts.

If the first insertable DNA fragment does not contain a regulatory region, a suitable regulatory region will need to be inserted operably linked to the structural gene, in order to provide an operable expression cassette. Similarly if no 3' germination sequence is provided at the insertion site to complete the expression cassette, a 3' termination sequence will have to be operably linked to the structural gene to be inserted.

Those skilled in the art are aware of numerous regulatory regions which have been characterized and could be employed in conjunction with methylotrophic yeasts. Exemplary regulatory regions include but are not limited to yeast regulatory regions selected from the group consisting of acid phosphatase, galactokinase, alcohol dehydrogenase, cytochrome c, alpha-mating factor and glyceraldehyde 3-phosphate dehydrogenase regulatory regions isolated from Saccharomyces cerevisiae; the primary alcohol oxidase (AOXI), dihydroxyacetone synthase DHAS, the p40 regulatory regions, and the HIS4 regulatory region derived from Pichia pastoris and the like. Presently preferred regulatory regions employed in the practice of the present invention are those characterized by their ability to respond to methanol-containing media, such regulatory regions selected from the group consisting of AOXI, DHAS, p40 and disclosed in EP-A-0183071.

The most preferred regulatory region for the practice of this invention is the AOXI regulatory region.

3' termination sequences may be utilized in the expression cassette or be part of the vector as discussed above. 3' termination sequences may function to terminate, polyadenylate and/or stabilize the messenger RNA coded for by the structural gene when operably linked to a gene. A few examples of illustrative sources for 3' termination sequences for the practice of this invention include but are not limited to the Saccharomyces cerevisiae, Hansenula polymorpha, and Pichia 3' termination sequences. Preferred are those derived from Pichia pastoris such as those selected from the group consisting of the 3' termination sequences of AOXI gene, DHAS gene, p40 gene and HIS4 gene. And particularly preferred is the 3' termination sequence of the AOXI gene.

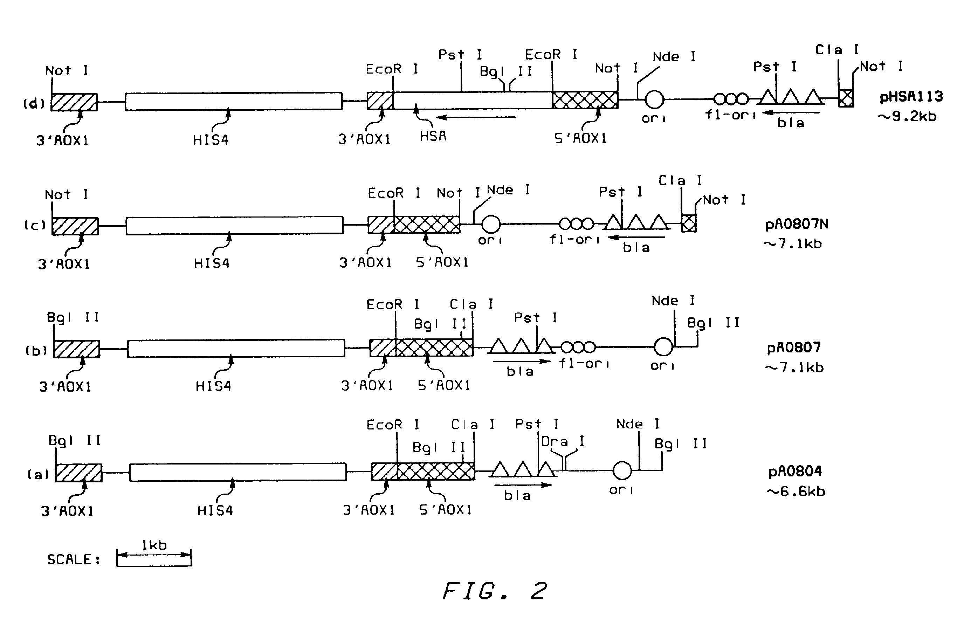

For the practice of the current invention either linear site-specific integrative vectors such as the BgIII fragments of the constructs shown in Figure 1 and 2 or plasmids such as provided in Figure 4 may be used.

The insertion of the HSA structural gene into suitable vectors may be accomplished by any suitable technique which cleaves the vector chosen at an appropriate site or sites and results in at least one operable expression cassette containing the HSA structural gene being present in the vector.

Ligation of HSA structural gene may be accomplished by any appropriate ligation technique such as utilizing T4 DNA ligase.

The initial selection, propagation, and optional amplification of the ligation mixture of the HSA structural gene and a vector is preferably performed by transforming the mixture into a bacterial host such as E. coli. (Although the ligation mixture could be transformed directly into a yeast host). Suitable transformation techniques for E. coli are well known in the art. Additionally, selection markers and bacterial origins of replication necessary for the maintenance of a vector is a bacterial host are also well known in the art.

The isolation and/or purification of the desired plasmid containing the HSA structural gene in an expression system may be accomplished by any suitable means for the separation of plasmid DNA from the host DNA.

Similarly the vectors formed by Ligation may be tested preferably after propagation to verify the presence of the HSA gene and its operable linkage to a regulatory region and a 3' termination sequence. This may be accomplished by a variety of techniques including but not limited to endonuclease digestion, gel electrophoresis, or endonuclease digestion-Southern hybridization.

Transformation of plasmids or linear vectors into yeast hosts may be accomplished by suitable transformation techniques including but not limited to those taught by Hinnen et al., Proc. Natl. Acad. Sci. 75, (1978) 1929; Ito et al., J. Bacteriol. 153, (1983) 163; Cregg et al Mol. Cell Biol. 5 (1985) pg. 3376; or Sreekrishna et al., Gene, 59 (1987) pg. 115. Preferable for the practice of this invention is the transformation technique of Cregg. It is desirable for the practice of this invention to utilize an excess of linear vectors and select for multiple insertions by Southern hybridization.

The yeast host for transformation may be any suitable methylotrophic yeast. Methylotrophic yeast include but are not limited to yeast capable of growth on methanol selected from the genera consisting of Hansenula, Candida, Kloeckera, Pichia, Saccharomyces, Torulopsis and Rhodotorula. A list of specific species which are exemplary of this class of yeasts may be found in C. Anthony, The Biochemistry of Methylotrophs, 269 (1982). Presently preferred are methylotrophic yeasts of the genus Pichia such as the auxotrophic Pichia pastoris GS115 (NRRL Y-15851). Auxotrophic methylotrophic yeasts are also advantageous to the practice of this invention for their ease of selection. It is recognized that wild type methylotrophic yeast strains may be employed with equal success if a suitable transforming marker gene is selected, such as the use of SUC2 to transform Pichia pastoris to a strain capable of growth on sucrose or an antibiotic resistance marker is employed, such as G418.

Transformed methylotrophic yeast cells can be selected for using appropriate techniques including but not limited to culturing previously auxotrophic cells after transformation in the absence of a biochemical product required (due to the cell's auxotrophy), selection for and detection of a new phenotype ("methanol slow"), or culturing in the presence of an antibiotic which is toxic to the yeast in the absence of a resistance gene contained in the transformant.

Isolated transformed methylotrophic yeast cells are cultured by appropriate fermentation techniques such as shake flask fermentation, high density fermentation or the technique disclosed by Cregg et al. High-Level Expression and Efficient Assembly of Hepatitis B Surface Antigen in the Methylotrophic Yeast, Pichia Pastoris 5 Bio/Technology 479 (1987).

### Examples

General information pertinent to the Examples:

### Strains

Pichia pastoris GS115 (his4) [NRRL Y-15851] Pichia pastoris KM71 (his4 aoxI:ARG4)
E.coli YMC9 (F⁻ λ⁻ endoAI hsds 17 SUPE44 thi I)
used for all plasmid constructions and preparations.
E.coli DH5αF' [(F', endAI hsd R17 (r⁻ₖ, mₖ⁺) Sup E 44, thi-I, λ⁻ recAI, gyr A96, rel AI, 080dlac Z-ΔM15, Δ(lac-YAargF)U169]

E.coli JM107 . endAI, gyrA96, thi, hsdR17, supE44, relAI,traD36 (r_{K}⁻, m_{K}⁺) Δ(lac proA,B)/F', proA,B, lacI^{q} ZΔM15. E. coli Y1088 [(ATCC no.37195); ΔlacU169 supE supF hsdR⁻ hsdM⁺ metB trpR tonA21 proC::Tn5 (pMC9). pMC9=pBR322-lacI^{Q}] was used for the amplification of the library for the purpose of DNA isolation, and preparation of plaque-purified phage stocks.
E. coli Y1090 [ATCC no.37197); ΔlacU169 proA± Δlon araD139 strA supF trpC22::Tn10 (pMC9)] was used as host for all immunological plaque screenings, and subsequent screening with various oligonucleotide probes.

### Buffers, Solutions and Media

The buffers, solutions and media employed in the following examples have the compositions given below:

| | Media, per liter |
|---|---|
| LB + amp | 10g yeast extract |
| | 20g tryptone |
| | 10g NaCl |
| | adjust to pH 7.5 with 5M NaOH |
| | 100mg ampicillin |
| LB | 10g yeast extract |
| | 20g tryptone |
| | 10g NaCl |
| | adjust to Ph 7.5 with 5M NaOH |
| MGY | 13.4g YNB without amino acids |
| | 400µg biotin |
| | 10g glucose |
| | 10ml glycerol |
| MM | 13.4g YNB without amino acids |
| | 400µg biotin |
| | 5ml methanol |
| LB agar plate | 1-2% agar in LB |
| LB top agar | 0.8% agar in LB |
| top agarose | 0.8% agarose in LB |
| LBM | LB + 10mM MgSO₄ |
| LBM/AMP | LBM + 50µg/ml ampicillin |
| H-top agar | 16g Bacto-tryptone |
| | 10g Bacto-yeast extract |
| | 5g NaCl in 1 liter H₂O |
| SM | 5.8g NaCl |
| | 2g MgSO₄ • 7H₂O |
| | 50ml 1M Tris • Cl, pH 7.5 |
| | 5ml 2% gelatin |
| SDR, 1 liter | 13.4g YNB |
| | 400 µg biotin |
| | 182g Sorbitol |
| | 10g dextrose |
| | 10g agar |
| | 50mg each of glutamine, methionine, lysine, leucine and isoleucine |
| | 2g histidine assay mix |
| SDHR, 1 liter | SDR + 40mg histidine |

### Buffers and Solutions

| | |
|---|---|
| TE Buffer: | 10mM Tris • Cl (pH 8.0) |
| | 1mM EDTA (pH 8.0) |
| | |
| High Salt Buffer: | 50mM Tris • Cl, pH 8.0 |
| | 10mM MgCl₂ |
| | 100mM NaCl |

### Restriction Digestion Buffers:

| | |
|---|---|
| HS buffer: | 50mM Tris • Cl (pH 7.5), 10mM MgCl₂, 100mM NaCl and 1 mM dithiothreitol |
| MS buffer: | 10mM Tris • Cl (pH 7.5), 10mM MgCl₂, 50mM NaCl and 1 mM dithiothreitol |
| LS buffer: | 10mM Tris • Cl (pH 7.5), 10mM MgCl₂ and 1 mM dithiothreitol |

### Ligation Buffer:

| | |
|---|---|
| 50mM | Tris • Cl (pH 7.4) |
| 10mM | MgCl₂ |
| 10mM | dithiothreitol |
| 1mM | ATP and |
| 100µg/ml | BSA. |

### Nick Translation (NT) Buffer:

| | |
|---|---|
| 50mM | Tris • Cl (pH 7.2) |
| 10mM | MgSO₄ |
| 0.1mM | dithiothreitol and |
| 1mM | EDTA |

### Phosphatase Buffer:

| | |
|---|---|
| 50mM | Tris • Cl (pH 9.0) |
| 1mM | MgCl₂ |
| 1mM | ZnCl₂ and |
| 1mM | Spermidine |

### Kinase Buffer:

| | |
|---|---|
| 50mM | Tris • Cl (pH 7.6) |
| 10mM | MgCl₂ |
| 5mM | dithiothreitol |
| 0.1mM | EDTA, and 0.1mM spermidine |

### Na₂CO₃/NaHCO₃ Buffer:

| | |
|---|---|
| .015M | Na₂CO₃ |
| .035M | NaHCO₃ |

### Blotto Buffer:

| | |
|---|---|
| 50g | nonfat dry milk |
| 1g | thimerosal |
| 100µl | anti foam A (Sigma) |
| 0.5ml | Tween 20 in 1X D-PBS (1l) (Dulbecco's Phosphate Buffered Saline, Gibco) |
| TBS, 1X | 150mM NaCl |
| | 10mM Tris • Cl, pH 7.5 |
| | |
| TBST | 1X TBS |
| | .05% Tween 20 |
| | |
| CaS | 1M Sorbitol |
| | 10mM CaCl₂ |
| | 10mM Tris • Cl, pH 7.5 filter sterilize |

### Example I

### Isolation of HSA-cDNA

A human liver λgtII-cDNA expression library (lot #2102) was purchased from Clontech Laboratories, Inc. This library had a titer of 9 x 10⁹ phage per ml, and was composed of 5.5 X 10⁵ independent, clear plaque phage isolates with an estimated insert size ranging from 0.15 to 1.8 kilobase pairs.

An aliquot representing 4 x 10⁵ independent phage isolates was screened for those which contained nucleotide sequences complimentary to probe #1. Probe #1 is a 19 bp oligonucleotide (shown in step 5 below) which includes the codons for the first six amino acids of the mature, secreted form a human serum albumin. Screening was performed as follows.

### Step.1. E. coli Transfection

E. coli Y1088, ATCC no. 37195 was suspended in liquid medium LBD composed of LB broth [5,0g/l yeast extract (Difco), 10.0g/l tryptone (Difco), 5.0g/l sodium chloride], supplemented with 0.2% D-glucose. A 50µl aliquot of this suspension was spread over a solit growth medium composed of 1.2% Agar Noble (Difco) in LB containing 50µg/ml ampicillin (LB+Amp). Bacterial colonies were allowed to grow overnight at 37°C. A few colonies were transferred to 10ml of LB-Amp media and was incubated at 30°C in a rotatory shaker set at 250 rpm in order to obtain an overnight culture to be used for screening of the library.

An aliquot of the λgtII-cDNA expression library was diluted one hundred fold in SM buffer [(50mM Tris • HCl, pH 7.5, 0.1M NaCl, 8.ImM MgSO₄, 0.01% (w/v) gelatin] . 4.4µl was mixed with 4.0ml of the overnight culture of E. coli Y1088, and the mixture was incubated at 30°C for 20 minutes. 0.2ml aliquots were added to 2.5ml soft agar medium maintained at 55°C [(0.7% Agar Noble (Difco) in LB] and was spread evenly over the bottom agar (LB containing 1.2% Agar Noble) in a petri dish with a diameter of 90mm. After 10 minutes at room temperature, the plates were placed in an incubator set at 43°C. The phage plaques were allowed to appear but not become confluent (usually about 4 to 6 hours).

### Step 2. Filter Lifts

Top agar containing plaques were overlayed with S&S Nitrocellulose filters (0.45µ pore size and 82mm in diameter). The filters on the agar were pierced in five spots with an 18 gauge needle on a syringe containing India ink in order to help in orienting the filters at a later stage. After one minute the filters were transferred with plaque side up on 3MM Whatmann filter paper saturated with 1.5M NaCl/0.5M NaOH. After 2 minutes the filters were blotted from the bottom side and transferred to a second 3MM Whatmann filter paper saturated with neutralizing solution (1.0M Tris • HCl, pH 8.0, 1.5M NaCl). After 8 minutes of neutralization, the filters were rinsed in 6XSSC (0.9 NaCl, 90mM sodium citrate, pH 7.0). Filters were blotted dry and were baked for 2 hrs. at 80°C in a vacuum oven.

### Step 3. Prehybridization and Hybridization With #1 Probe and Autoradiography

Plaques which contained cDNA for human serum albumin were identified by the hydridization of their DNA with radioactivly-labeled oligonucleotides which were selected for their nucleotide sequence complementarity with human serum albumin cDNA, but not complementarity with either bacterical or transforming vector DNAs.

### A. Preparation of Labeled Oligonucleotide Probes

Radioactive oligonucleotide probes were prepared by mixing approximately 100ng of a given oligonucleotide with 100µCi of [-³²P] ATP in 10µl of a buffer containing 70 mM tris-HCl (pH 7.6), 10 mM MgCl₂, 1.0 mM KCI, 5.0 mM dithiothreitol, 1.0 mM spermidine. Ten units of T4 polynucleotide kinase was added and the mixture was incubated at 37°C for 30 minutes. The enzyme was inactivated by a 5 minute incubation at 65°C, and the labeled oligonucleotide was added to the hybridization solution without any purification from the reaction mixture.

### B. Prehybridization

The filter lifts containing phage DNA were incubated for 3-16 hours in a solution composed of 0.9M NaCl, 6.0mM Na₂EDTA, 19.8mM Tris • HCl (pH 8.0), 0.1% Ficoll, 0.1% polyvinylpyrrolidone, 0.1% bovine serum albumin, 0.1% SDS and 10% Dextran sulfate. The temperature of incubation was 37°C. Ten milliliters of solution was used per filter.

### C. Hybridization

The prehybridized filters were hybridized in the same solution as prehybridization, but containing radioactivlylabeled oligonucleotide #1 at a concentration of 2ng/ml. Incubation was for 20-48 hr. at 37°C using 2ml of solution per filter.

### D. Washing

Filters were washed four times (20 minutes per wash) at room temperature in 0.9M NaCl, 90mM sodium citrate (pH 7.0), and once at 45°C (1 hour) in the same buffer but containing 0.1% SDS. 10ml volumes of solutions were used for washing each filter.

### E. Autoradiography

Filters were air dried and placed in a x-ray film exposing cassette adjacent to an x-ray film (Kodak XAR-2 or XAR-5). Cassettes were incubated at -80°C for 20-48 hrs.

### Step 4. Immunoscreening for HSA Protein Positive Plaques

The cloning site in the λgtII vector was within the β-galactosidase coding region. Since IPTG (isopropyl β-D-thiogalactoside) induces expression of the β-galactosidase gene in the λgtII vector, it may also be used to induce expression of genes that are cloned in-frame with respect to β-galactosidase and produce β-galactosidase fusion-proteins. Therefore, whale or partial HSA genes in-frame and in the correct orientation should yield immunoreactive material. The E.coli strain Y1090 ATCC No. 37197 was used for the detection of HSA expression-positive plaques. Infection with phage and plating procedures were as described above for the E. coli strain Y1088. After the plaques were approximately lmm in diameter, nitrocellulose filters that had been previously soaked in 10mM (IPTG) and dried were placed on the top agar. Plates were incubated at 43°C for 4-6 hours. The filters were then removed from the plates and immersed in a blocking solution composed of 1% gelatin, 0.05% Brij 58 in TBST. Filters were incubated at room temperature with gentle rocking for 0.5-16 hours. When plaque lifts were desired from the same plates for hybridization with oligonucleotides, the plates were returned to the 43°C incubator for 2 hours prior to overlaying the nitrocellulose filters.

The IPTG filters were removed from the blocking solution and plaques expressing immunoreactive HSA were identified using a 1:1000 dilution of goat anti-HSA (nephelometric grade, Atlantic Antibodies, Catalog number 001-11, Scarborough, ME) in blocking solution. After incubation for 1 hour at room temperature, filters were washed five times in TBST, for 10 minutes per wash. All incubations and washings were performed with gentle rocking. Binding of anti-HSA antibodies was detected by incubation with a 1:1000 dilution (0.5 µg/ml) of alkaline phosphatase-conjugated rabbit antigoat IgG (Kirkegaard and Perry Laboratories, Gaithersburg, MD) in blocking solution for 1 hour at room temperature. After washing as above, filters were incubated in the phosphatase substrate system composed of nitrobluetetrazolium and 5-bromo-4-chloro-3-indolyl phosphate (Kirkegaard and Perry Laboratories) in 0.1 M Tris buffer for 10-30 minutes at room temperature. A stable purple precipitate was deposited at the reaction site in the membrane. The reaction was stopped by washing filters in water, and the filters were dried in air.

In some experiments ¹²⁵I-protein G was used for identification of positive plaques. In this case, the filters were incubated for 4 hours in a solution of TBS (TBST without Tween-20) containing 0.3µCi of ¹²⁵I-protein G per ml, following incubation with anti-HSA antibody and subsequent washes, as described above. Filters were washed twice in BTBS (TBS containing 0.05% Brij 58) followed by two additional washes in TBS. All washing were done at 10 minute intervals with gentle rocking at room temperature. Autoradiography was performed as described above.

### Step5. Plaque Hybridization with Oligonucleotides #2, #3, M1 and M2

Purified plaques were screened with the following oligonucleotides:
- #1:: 5'-CCTCACTCTTGTGTGCATC-3'
- #2:: 5'-CCACTTCGGCAATGCAGTGGGATTTTTCCAACAGAGG-3
- #3:: 5'-CCCTCCTCGGCAAAGCAGG-3
- M1:: 5'-GAAATAAAGGTTACCCACTTCAT-3'
- M2:: 5'-CCCACTTCATTGTGCCAAAG-3'

The #1 probe hybridizes to 10 nucleotides corresponding to the first six amino acids of the mature HSA protein. The #2 probe is complementary to the nucleotides which correspond to amino acids #281 to #292 of the mature HSA protein, and are upstream from a single PstI restriction endonuclease site within the HSA-cDNA sequence. The #3 probe can be used for the detection of clones which include nucleotides for the amino acids #565 to #571 of the mature HSA protein. The above three oligonucleotides were chosen, because there were no nucleotide variations in the published HSA cDNA sequences within those regions.

Potential probes were screened for lack of sequence homology to the known sequences in λgtII vector, and E. coli DNA. This screening was necessary in order to decrease non-specific sites of hybridization. Based on this analysis the probe M2 could be expected to exhibit a low level of binding to vector sequences. Since the M2 probe includes nucleotides complementary to ten bases upstream from the ATG start codon, any clone hybridizing under high stringency conditions would be expected to contain all or most of the ten bases and hence, the entire HSA coding sequence.

### Example II

### Cloning of Human Serum Albumin CDNA into M13mP18 for Sequence Analysis

### Step 1. Preparation of M13mp18 Clonging Vector

M13mp18 (New England Biolabs) replicative form DNA (4µg) was digested to completion with 40 units of EcoRI in 50µl of high salt buffer at 37°C for 1 hour. The digested sample was heated at 70°C for 5 minutes to inactivate the enzyme. Calf intestinal alkaline phosphatase (CIAP, 1 unit) was added and the mixture was incubated at 50°C for 1 hour. The reaction was terminated by addition of 1/10 volume of 500 mM EGTA (pH 8). The CIAP was inactivated by heating at 65°C for 45 minutes. The reaction mixture was extracted with an equal volume of TE-saturated phenol followed by chloroform extraction. The linearized M13mp18 in the aqueous phase was precipitated by adding 3 volumes of 3M sodium acetate and absolute ethanol mixture (1:30, V/V). The mixture was allowed to stand at -20°C overnight. The DNA was recovered by centrifugation for 15 minutes at 14,000 X g at 4°C. The pellet was vacuum dried and redissolved in 100µl of TE.

### Step 2. Preparation of HSA cDNA Insert and ligation with M13mp18 Vector

### A. Preparation of High Titer Plate Lysates

Recombinant phages positive for the antibodies and oligonucleotide screenings as described in Example I were amplified for preparation of phage DNA. The plating cells were prepared from an overnight culture of E. coli Y1088 in 40 ml of LBM+AMP. Cells were harvested by centrifugation at 3,000 X g for 15 minutes at room temperature and the pellet was resuspended in 15-16 ml of 10 mM MgSO₄. Individual plaques from LB plates (prepared as described in Example I) in the form of agar plugs were suspended in 0.1 ml of E. coli Y1088 plating cells. After standing at room temperature for 10 minutes, 2.5 ml of LBM and 2.5 ml of LB top agar at 55°C was added. The mixture was poured onto prewarmed (42°C) LB agar plates. After about 10 minutes at room temperature, the plates were inverted and incubated at 42°C overnight. On the next day, the phage particles were recovered by flooding the plates with 3 ml of SM. One drop of chloroform was added and the tube was vortexed. The content was centrifuged at 4,000 X g for 10 minutes to remove the cell debris. The supernatant (high-titer lysate) was stored at 4°C.

### B. Titering of the High-titer Lysate

lµl and 10ul aliquots from the 10⁻⁴ dilution of the high-titer lysate in SM were mixed with 0.3 ml of E. coli Y1088 plating cells. After incubation at 37°C for 10 minutes, 3 ml of LB top agar prewarmed at 55°C was added. The mixture was poured onto LB plates and incubated at 42°C overnight. The number of plaque forming units (pfu) per ml lysate was recorded as the titer.

### C. Preparation of Phage DNA by Plate Lysate Method

About 10⁶ pfu were plated out as described above for the titering except that 6 ml of top agarose was used in the 150 mm diameter petri dish. After confluent lysis of the plating bacteria, the phage was extracted from the top agarose into SM. The phage DNA was then isolated from the supernatant by using Lambdasorb Phage Adsorbant (Promega Biotec, Madison, WI) according to the suggested protocol of the manufacturer.

### D. Ligation Reaction with M13mp18

HSA-λgtll recombinant DNA (4-5µg) was digested with 20 units of EcoRI in 30µl of high salt buffer for 3 hours at 37°C. After digestion, the reaction mixture was processed further as described in step 1. The total mixture was ligated using the T4 ligase with the EcoRI-CIAP treated M13mp18. A typical ligation reaction (final volume 10µl) contained 4-5µg of total DNA and 0.8µg of M13mp18 in 1 X ligation buffer (50mM Tris-HCl, pH 7.6, 10mM MgCl₂, 1 mM ATP, 1mM DTT, 5% (W/V) polyethylene glycol-8000). Ligation was performed at 15°C overnight using 1-2 units of T4 DNA ligase.

### Step 3. Transformation of Competent Cells

Aliquots of the ligation mixture (1-5µl) were mixed with 300µl of E. coli JM107 competent cells [Mandel et al., J. Mol. Biol. 53, 154 (1970)] or 100µl of E. coli DH5αF' frozen competent cells (BRL) and kept on ice for 40 minutes. Uptake of DNA was induced by heat shock at 42°C for 2 minutes. The transformed cells were then returned to ice. A plating mixture containing 200µl of freshly prepared E. coli JM107 in exponential phase, 40µl of X-gal in 2% dimethylformamide, 100µl of 10 mM IPTG and 3ml of H-top agar at 55°C was added. The transformation mixture was poured onto LB agar plates. The plates were incubated overnight at 37°C for plaque formation.

### Step 4. Preparation of M13 ssDNA Templates and DNA Sequence Analysis

Colorless plaques containing recombinant M13 phage were picked and amplified in E.coli JM107 in 1.5 ml of 2XYT at 37°C for 5-6 hours with vigorous shaking. After amplification, the cultures were spun down at 14,000g for 1 min. to separate the cells from the supernatant. The cell pellets were used for preparation of the double-stranded replicative form of M13, while the supernatants were used for the isolation of single-stranded DNA templates. M13 ds RF was isolated using the alkaline lysis method of Birnboim and Doily, Nucl. Acids Research 7:1513 (1979). M13 ssDNA templates were isolated from the culture supernatants by polyethylene glycol precipitation and phenol extraction. Briefly, Iml of the culture supernatant ml) was mixed with 200µl of 20% polyethylene glycol-6000 in 2.5 M NaCl. After leaving the mixture at room temperature for 15 minutes, the M13 phage particles were recovered by centrifugation at 14,000 X g for 5 minutes. The phage pellet was resuspended in 100 µl TE followed by extraction with buffer-saturated phenol. The aqueous phase containing the M13 ssDNA was mixed with 3 volumes of sodium acetate and ethanol mixture. After chilling at - 20°C overnight, the DNA precipitate was recovered by centrifugation at 14,000 X g for 10 minutes at 4°C. The dried DNA was dissolved in 50µl TE and 5µl was used for the sequencing reaction. DNA sequencing was performed by the dideoxynucleotide chain termination method of Sanger et al., Proc. Natl. Acad. Sci., 74:5463 (1977). The complete sequence of HSA 13 is shown at Table 1.

### Example III

### Creation of pA0807N

The pA0804 plasmid is available in an E. coli host from the Northern Regional Research center of the United States Department of Agriculture, Peoria, Illinois (accession number NRRL B-18114). PA0804 is recoveredby isolating the plasmid DNA, digesting with EcoRI, gel electrophoresing to recover the ~7.5 Kb fragment, which is linear pA0804 cut at its unique EcoRI site. Plasmid pA0807N was constructed starting from pA0804, pBR322 and bacteriophage fl DNA as follows.

### Step 1. Preparation of fl-ori DNA

fl bacteriophage DNA (50µg) was digested with 50 units of RsaI and DraI at 37°C for 4 hours in 200µl of MS buffer to release the ~458 bp DNA fragment containing the fl origin of replication (ori). The digestion mixture was extracted with an equal volume of phenol:chloroform (V/V) followed by extracting the aqueous layer with an equal volume of chloroform. Finally, the DNA in the aqueous phase was precipitated by adjusting the NaCl concentration to 0.2M and adding 2.5 volumes of absolute ethanol. The mixture was allowed to stand on ice (4°C) for 10 minutes and the DNA precipitate was collected by centrifugation for 30 minutes at 10,000 xg in a microfuge at 4°C. The DNA pellet was washed 2 times with 70% aqueous ethanol. The washed pellet was vacuum dried and dissolved in 25µl of TE buffer. This DNA was electrophoresed on 1.5% agarose gel and the gel portion containing the ~458 bp fl-ori fragment was excised out and the DNA in the gel was electroeluted into 500µl of 5mM EDTA pH 8.0). The DNA solution was phenol:chloroform extracted as detailed above and the DNA precipitate was dissolved in 25µl of TE buffer (fl-ori fragment).

### Step 2. Cloning of fl-ori into DraI Sites of pBR322

pBR322 (2µg) was partially digested with 2 units DraI in 20µl of MS buffer at 37°C for 10 minutes. The reaction was terminated by phenol:chloroform extraction followed by precipitation of DNA as detailed in step I above. The DNA pellet was dissolved in 20µl of TE buffer. About 100 ng of this DNA was ligated with 100 ng of fl-ori fragment (step I) in 20µl of ligation buffer by incubating at 14°C overnight with I unit of T4 DNA ligase. The ligation was terminated by heating at 70°C for 10 minutes and then used to transform E. coli strain YMC9 (Maniatis, et al.) to obtain pBRfl-ori which contains fl-ori cloned into the DraI sites (nucleotide positions 3232 and 3251) of pBR322.

### Step 3. Creation of pA0807

pBRfl-ori (10µg) was digested for 4 hours at 37°C with 10 units each of PstI and NdeI. The digested DNA was phenol:chloroform extracted, precipitated and dissolved in 25µl of TE buffer as detailed in step I above. This material was electrophoresed on a 1.2% agarose gel and the NdeI - PstI fragment (approximately 0.8 kb) containing the fl-ori was isolated and dissolved in 20µl of TE buffer as detailed in Step I above. About 100 ng of this DNA was mixed with 100 ng of pA0804 that had been digested with PstI and NdeI and phosphatase treated. This mixture was ligated in 20µl of ligation buffer by incubating for overnight at 14°C with I unit of T4 DNA ligase. The ligation reaction was terminated by heating of 70°C for 10 minutes. This DNA was used to transform E. coli strain YMC9 to obtain pA0807.

### Step 4. Conversion of the Two BglII Sites in pA0807 to NotI Sites to Create pA0807N

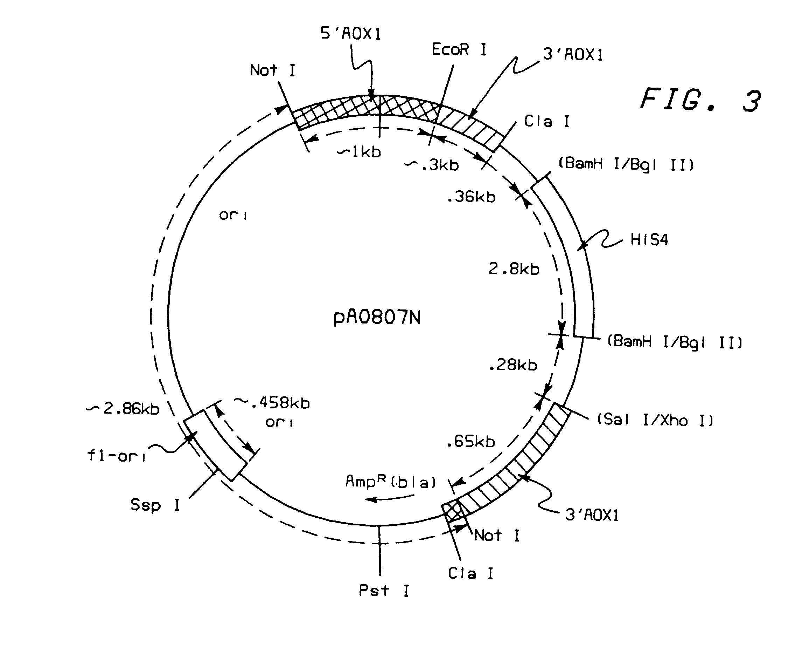

pA0807 (10 µg) was digested with 10 units of BglII for 4 hours at 37°C in 50µl of HS buffer. The BglII cohesive ends were filled in by incubating the BglIII cleaved DNA (10µg) in 50 ul of NT buffer with 5 units of the Klenow fragment of DNA polymerase at room temperature for 30 minutes. This mixture was phenol:chloroform extracted and the DNA was recovered as described in step I above. The DNA pellet was dissolved in 25 ul of TE buffer. This DNA was mixed with 50 ng (I ul) of phosphorylated NotI linker (pGCGGCCGC) obtained from New England Biolabs, 40ul of 5x ligation buffer, 129 ul water and 5 units of T4 DNA ligase. This mixture was incubated overnight at 14°C. Ligation was terminated by heating to 70°C for 10 minutes. Following this the ligation mixture was digested with 10 units of NotI after adjusting the solution to HS buffer condition. The DNA was precipitated after phenol:chloroform extraction as detailed in step I above. The precipitate was dissolved in 50 ul of TE buffer and electrophoresed on a 0.9% agarose gel. The DNA fragments lower band corresponded to the migration position of the fragment containing pBR322 portion and fl-ori and the upper band corresponded to the remaining portion of pA0807. i.e., 5'AOXI, 3'AOXI and HIS4) were isolated from the gel by using the protocol described in step I above. The gel purified DNA fragments were dissolved in 10 ul of TE buffer. The DNA fragment representing the linear site specific integrative vector was phosphatased by incubating for 30 minutes with 2 units of CIAP at 37°C in 200µl of phosphatase buffer. The phosphatased DNA was phenol:chloroform extracted and precipitated as described in step I. This DNA was mixed with the upper band DNA representing the rest of the pA0807 plasmid (see above) and ligated overnight at 4°C with 5 units of T4 DNA ligase in 30 ul of ligation buffer. The ligation mixture was heated for 10 minutes at 70°C, cooled on ice and a 10 ul aliquot was used to transform E. coli YMC9 to obtain pA0807N. The structure of pA0807N is shown in Figure 3.

### Example IV

### Construction of Pichia pastoris HSA Expression Vectors

### Step I. Recovery of HSA Fragment

The HSA gene corresponding to about 2.0 Kb was released from mp18-HSA13 replicative form DNA (see example II) by EcoRI digestion. About 1 µg of plasmid mp18-HSA13 was digested at 37°C for 2 hours with 5 units of EcoRI in 20 ul of HS buffer. The reaction was terminated by diluting to 50 ul with dH₂0 immediately extracted with phenol:chloroform, and precipitated as detailed in Step I of Example III. The DNA precipitate was dissolved in 10 ul of water and stored at -20°C later use. The HSA gene was inserted into vectors pTHFKΔ and pA0807N at their EcoRI sites to obtain the Pichia pastoris HSA expression plasmids pHSA13 and pHSA113.

### Step 2. Vector Manipulations for Insertion of HSA Gene

About 10 µg each of pTHFKΔ (Figure 4) and pA0807N were digested with 10 units of EcoRI in 100ul of HS buffer for 16 hours at 37°C. The reaction mixture was adjusted to alkaline phosphatase buffer conditions and treated with 10 units of CIAP in 200 ul reaction volume for 30 minutes at 37°C. Phosphatase treatment was terminated by phenol:chloroform extraction and the DNAs were precipitated and dissolved in TE buffer at a final concentration of 100 ug/ml as detailed in step I of example III.

### Step 3. Insertion of HSA Gene into Expression Vectors

About 100 ng each of EcoRI cut and CIAP treated vectors pTHFKΔ and pA0807N (step 2, example IV) were mixed with approximately 100 ng each of EcoRI digested mp18-HSA13 (Step 1, Example IV) in 20 ul of ligation buffer and ligated with 2 units of T4 DNA ligase at 4°C for 16 hours. Ligation was terminated by heating to 70°C for 10 minutes and used to transform E. coli strain DG75' to obtain plasmids pHSA13 and pHSA113.

### Example V

### Transformation of Pichia pastoris with pHSA113 and pHSA13

### Step 1. pHSA113 Vector Preparation

About 20 ug of pHSA113 was digested for 18 hours at 37°C in 200 ul of HS buffer with 50 units of NotI. About 20 ul of this mixture was directly used for transformation of Pichia pastoris GS115 (his4) deposited with the Northern Regional Research Center of the United States Department of Agriculture, accession number NRRL Y-15851. The remaining approximately 180 ul of the NotI cleaved pHSA113 was phenol: chloroform extracted and precipitated as detailed in step I of example III. The DNA precipitate was dissolved in 20 ul of CaS solution and was also used for transformation of GS115. The NotI cleaved pHSA113 can integrate into a Pichia locus. Because the NotI fragment form pHSA113 also carries the histidinol dehydrogenase gene of Pichia the resulting transformants can be readily selected based on the His⁺ phenotype.

Pichia strain KM71 (his4, aoxl:ARG4) was transformed for His⁺ with 10 ug of pHSA 13. In addition to HIS4 this plasmid carries an autonomously replicating sequence ARSI. It can replicate within Pichia in the autonomous form. Such transformants will be referred to as "autonomous transformants". The reason for using this strain is that KM71 is "methanol-slow" due to the disruption of AOXI by ARG4 and has been shown to express higher levels of β-galactosidase when lacZ is placed under the control of the AOXI promoter than the methanol-normal strain GS115. For negative control KM71 was also transformed with pYJ30, NRRL B-15890, a plasmid also containing His4.

### Step 2. Cell Growth

Pichia pastoris GS115 (NRRL Y-15851) was inoculated into about 10 ml of YPD medium and shake cultured at 30°C for 12-20 hours. 100 ml of YPD medium was inoculated with seed culture to give an OD₆₀₀ of about 0.001. The medium was cultured in a shake flask at 30°C for about 12-20 hours. The culture was harvested when the OD₆₀₀ was about 0.2-0.3 (after approximately 16-20 hours) by centrifugation at 1500 g for 5 minutes using a Sorvall RC5C.

### Step 3. Preparation of Spheroplasts

The cells were washed once in 10 ml of sterile water, and then centrifuged at 1500 g for 5 minutes. (Centrifugation is performed after each cell wash at 1500 g for 5 minutes using a Sorvall RT6000B unless otherwise indicated). The cells were then washed once in 10 ml of freshly prepared SED, once in 10 ml of sterile 1 M sorbitol, and finally resuspended in 10ml of SCE buffer. 7.5 µl of 3mg/ml Zymolyase (100,000 units/g obtained from Miles Laboratories) was added to the cell solution. The cells were then incubated at 30°C for about 10 minutes. (A reduction of 60% in OD₆₀₀ can be utilized as a correct time and concentration marker). The spheroplasts were washed once in 10 ml of sterile 1 M sorbitol by centrifugation at 700 g for 5-10 minutes. (The time and speed for centrifugation may vary; centrifuge enough to pellet the spheroplasts but not so much they rupture from the force). 10 ml of sterile CaS was used as a final cell wash, and the cells were centrifuged again at 700 g for 5-10 minutes and resuspended in 0.6 ml of CaS.

### Step 4. Transformation

GS115 cells were transformed with 10 µg of the linear HSA vectors using the spheroplast transformation technique of Sreekrishna et al. in Gene 59, 115-125 (1987). DNA samples were added (up to 20 µl volume) to 12 x 75 mm sterile polypropylene tubes. (DNA should be in a suitable buffer, such as TE buffer). 100 µl of spheroplasts were added to each DNA sample and incubated at room temperature for about 20 minutes. I ml of PEG solution was added to each sample and incubated at room temperature for about 15 minutes and centrifuged at 700 g for 5-10 minutes. SOS (150 µl) was added to the pellet and incubated for 30 minutes at room temperature. Finally, 850 µl of 1 M sorbitol was added.

### Step 5. Regeneration of Spheroplasts

A bottom agar layer of 20 ml of regeneration agar SDR was poured per plate at least 30 minutes before transformation samples were ready. In addition, 8 ml aliquots of regeneration agar were distributed to 15 ml conical bottom Corning tubes in a 45°C bath during the period that transformation samples were in SOS. Aliquots of 50, 250 or 800 µl of the transformed sample was added to the 8 ml aliquots of melted regeneration agar held at 45°C and poured onto plates containing the solid 20 ml bottom agar layer. The plates were incubated at 30°C for 3-5 days.

### Step 6. Selection of Transformants

Transformants were selected for by culturing on SDR, a media lacking histidine. Cultures which grew in the absence of histidine were additionally screened for the "methanol slow" phenotype (indicating site selective integration). The transformed GS115 cells showing evidence of both phenotypes were then cultured and assayed for the production of HSA.

### Example VI

### Methanol Induced Expression of HSA in GS115/pHSA13 Autonomous Transformants

KM71 strains transformed with plasmid pHSA13 and negative control KM71 transformed with pYJ30 were grown in 10 ml cultures on MGY to an optical density of 8.00 at 600 nm and then shifted to MM medium. After incubation on MM for 3 days, cells were harvested by centrifugation and media supernatant was adjusted to 1 mM phenylmethylsulfonyl fluoride (PMSF) and stored frozen for HSA analysis (Supernatant M). The cells were suspended in 500 ul breaking buffer containing 1 mM PMSF and vigorously vortexed with glass beads for a total of 4 minutes with intermittent periods on ice. Following this the samples were centrifuged in a microfuge at 10,000 xg for 10 minutes at 4°C and the clear supernatant solution was separated from the pellet and designated as Supernatant I. The pellet was extracted with 500 ul breaking buffer containing 6M urea and this extract was designated as Supernatant II. The various supernatants were analyzed for HSA by PAGE immunoblot as described in Methods in Enzymology, Vol. 152 (1987), "Guide to Molecular Cloning Techniques", as well as quantitative HSA-ELISA. The HSA-ELISA procedure developed for this purpose is given in Example VIII. Supernatant I contained the highest level of HSA compared to other fractions as determined by HSA-ELISA (Table 3) (HSA was present predominantly in the soluble fraction as assessed by PAGE-electroimmunoblotting).

**Table 3**

| HSA Concentration in Supernatant I Prepared from KM71 Transformants | | |
|---|---|---|
| Transformant | ng HSA/mg protein | HSA % |
| KM71/pYJ30-1 | <1 | <0.0001% |
| KM71/pHSA13-1 | 8914 | 0.89% |
| KM71/pHSA13-2 | 15777 | 1.6% |
| KM71/pHSA13-4 | 5648 | 0.56% |
| KM71/pHSA13-6 | 88885 | 8.9% |

### Example VII

### Methanol Regulated Expression of HSA in GS115/pHSA113 Integrative Transformants

Several thousand His⁺ transformants of GS115 obtained using NotI cleaved pHSA113 were pooled and an aliquot was inoculated into 10 ml of MGY and grown to saturation. At this point cells were switched to MM and incubated at 30°C on a shaker. After 2 days on MM, cells were harvested and supernatant I was prepared and analyzed for HSA expression as described in example VI. The expression level of HSA was ∼0.1% of soluble protein.

The His⁺ transformant pool was also screened for "methanol slow" transformants by replica plating colonies on MD plates onto MM plates. Several His⁺ -methanol slow transformants were grown on MGY and shifted to MM. Cell extracts were prepared and analyzed for HSA expression levels as described in Example VI. Levels of HSA were detected up to 20 mg/I, or 2% of total soluble cellular potein.

### Example VIII

### HSA ELISA

50µl of 1:500 goat-anti-HSA antibodies in Na₂C0₃/NaHC0₃ buffer, pH 9.5, were placed in wells of 96 well ELISA plates (Corning) and incubated for 1 hr. at 37°C. They were washed 2 X with TBST, 2 X with dH₂0, 200µl of blotto buffer were added to each, and they were then incubated overnight at 37°C. After incubation, they were once again washed 3X with TBST, 2X with dH₂0, and 50µl of sample was added to each (stock HSA solution = 5X10⁷ ng/ml; standard solutions = 2-14 ng/ml). The samples were rotated for two hours at room temperature, then washed 5X with TBST, 2X with dH₂0, and 50µl of a 1:2000 dilution of horseradish peroxidase-conjugated goat anti-HSA antibodies (Cooper Biomedical, Inc.) in blotto buffer (5µl/10ml) were added. The plates were again shaken for I hr. at room temperature, then washed 3X with TBST, 3X with dH₂0 and 100µl of ABTS (ABTS peroxidase substrate solution, Kirkegaard and Perry Labs., Inc.) warmed to room temperature was added. Finally, the samples were shaken for 20 min. at room temperature, 100µl of 2% oxalic acid was added to each to stop the reaction, and absorbance at 405nm was read.

The following pages 38 - 42 of the description refer to preferred embodiments (emb.) of the description.
1. A process for the production of HSA comprising
   a) transforming a methylotrophic yeast with at least one vector having at least one expression cassette containing a structural gene for HSA, operably linked to a regulatory region and a 3' termination sequence; and thereafter
   b) culturing the resulting transformed yeast strain to obtain the production of said HSA protein.
2. The process of emb. 1 wherein said vector is selected from plasmids and linear integrative site-specific vectors.
3. The process of emb. 2 wherein said linear integrative site-specific vector contains the following serial arrangement:
   a) a first insertable DNA fragment,
   b) at least one marker gene, and at least one expression cassette containing a structural gene for HSA, operably linked to a regulatory region and a 3' termination sequence, and
   c) a second insertable DNA fragment;
   wherein the order of the marker gene and cassette of component (b) may be interchanged.
4. The process of emb. 3 wherein the first insertable DNA fragment and the second insertable DNA fragment are derived from the DNA sequence of a gene isolated from Pichia pastoris and selected from AOX1, p40, DHAS and HIS4.
5. The process of emb. 3 wherein said expression cassette comprises
   a) a regulatory region selected from AOX1, p40, DHAS and HIS4, isolated from Pichia pastoris, acid phosphatase, galactosidase, alcohol dehydrogenase, cytochrome c, alpha-mating factor and glyceraldehyde 3-phosphate dehydrogenase isolated from Saccharomyces cerevisiae operably linked to
   b) a structural gene for HSA, operably linked to
   c) a 3' termination sequence from Pichia pastoris selected from the 3' termination sequence isolated from the AOX1 gene, p40 gene, DHAS gene and HIS4 gene.
6. The process of emb. 3 wherein said marker gene is selected from HIS4 and ARG4, isolated from Pichia pastoris, SUC2 isolated from Saccharomyces cerevisiae and G418^{R} gene of Tn903 and Tn601.
7. The process of emb. 3 wherein said vector comprises
   a) a first insertable DNA fragment which is about one kilobase of the 5' AOX1 regulatory region isolated from Pichia pastoris operably linked to
   b) a structural gene for HSA, operably linked to
   c) the 3' termination sequence of AOX1 isolated from Pichia pastoris ligated to
   d) at least one marker gene which is HIS4 isolated from Pichia pastoris ligated to
   e) a second insertable DNA fragment which is about 0.65 kilobases of the 3' AOX1 termination sequence.
8. The process of emb. 2 wherein the plasmid comprises an autonomously replicating DNA sequence and a marker gene.
9. The process of emb. 8 wherein said plasmid comprises
   a) the 5' AOX1 regulatory region isolated from Pichia pastoris operably linked to
   b) a structural gene for HSA, operably linked to
   c) the 3' termination sequence of AOX1 isolated from Pichia pastoris ligated to
   d) at least one marker gene
   e) a second DNA fragment which is about a 0.19 kilobase sequence of an autonomous replicating DNA sequence.
10. The process of emb. 8 wherein said marker gene is as defined in claim 6; preferably wherein said marker gene is HIS4.
11. A linear integrative site-specific vector comprising the following serial arrangement
   a) a first insertable DNA fragment,
   b) at least one marker gene and at least one expression cassette containing a structural gene for HSA, operably linked to a regulatory region and a 3' termination sequence, and
   c) a second insertable DNA fragment;
   wherein the order of the marker gene and cassette of component (b) may be interchanged.
12. The vector of claim 11, wherein said vector is defined as in any of emb.s 4 - 7.
13. A plasmid comprising the following
   a) an autonomously replicating sequence capable of replication in methylotrophic yeasts
   b) at least one marker gene
   c) at least one expression cassette containing a structural gene for HSA operably linked to a regulatory region and a 3' termination sequence.
14. The plasmid of emb. 19 wherein said expression cassette and said marker gene are as defined in claims 5 and 6.
15. The vector pHSA13.
16. Methylotrophic yeast transformed with at least one vector containing at least one expression cassette comprising a regulatory region operably linked to structural gene for HSA, operably linked to a 3' termination sequence.
17. The methylotrophic yeast of emb. 16 wherein the yeast is Pichia pastoris.
18. The methylotrophic yeast of emb. 16 wherein the yeast is Pichia pastoris strain GS115.
19. The methylotrophic yeast of any of emb.s 16 - 18 wherein said vector is at least one linear integrative site-specific vector as defined in any of claims 3 - 7.
20. Pichia pastoris GS115/pHSA113.
21. Pichia pastoris GS115 transformed as in emb. 18 or 19 wherein said GS115 is transformed with more than one copy of said linear integrative site-specific vector.
22. The yeast of any of emb.s 16 - 18 wherein said vector is at least one plasmid as defined in claim 13 or 14.
23. Pichia pastoris GS115/pHSA113.
24. A nucleotide sequence coding for HSA wherein said sequence is recited in Table 1.

## Claims

1. A process for the secretion of HSA in Pichia pastoris comprising
(a) transforming a methylotrophic yeast of Pichia pastoris with at least one vector having at least one expression cassette containing a structural gene for HSA which includes the secretion signal sequence, operably linked to a regulatory region and a 3' termination sequence; and thereafter
(b) culturing the resulting transformed yeast strain to obtain the secretion of said HSA protein.

2. The process of claim 1 wherein said vector is selected from plasmids and linear integrative site-specific vectors.

3. The process of claim 2 wherein said linear integrative site-specific vector contains the following serial arrangement:
(a) a first insertable DNA fragment,
(b) at least one expression cassette containing a structural gene for HSA which includes the secretion signal sequence, operably linked to a regulatory region and a 3' termination sequence, and
(c) a second insertable DNA fragment.

4. The process of claim 3 wherein the first insertable DNA fragment and the second insertable DNA fragment are derived from the DNA sequence of a gene isolated from Pichia pastoris and selected from AOX1, p40, DHAS, and HIS4.

5. The process of claim 3 wherein said expression cassette comprises
(a) a regulatory section selected from AOX1, p40, DHAS, and HIS4, isolated from Pichia pastoris, acid phosphatase, galactokinase, alcohol dehydrogenase, cytochrome c, alpha-mating factor and glyceraldehyde 3-phosphate dehydrogenase isolated from Saccharomyces cerevisiae operably linked to
(b) a structural gene for HSA which includes the secretion signal sequence operably linked to
(c) a 3' termination sequence isolated from AOX1 gene, p40 gene, DHAS gene and HIS4 gene.

6. The process of claim 3 wherein said vector comprises
(a) a first insertable DNA fragment which is about 1 kilobase of the 5' AOX1 regulatory region isolated from Pichia pastoris operably linked to
(b) a structural gene for HSA which includes the secretion signal sequence operably linked to
(c) a 3' termination sequence of AOX1 isolated from Pichia pastoris ligated to
(d) at least one marker gene which is HIS4 isolated from Pichia pastoris ligated to
(e) a second insertable DNA fragment which is about 0.65 kilobases of the 3' AOX1 termination sequence.

7. The process of claim 2 wherein said plasmid comprises
(a) the 5' AOX1 regulatory region isolated from Pichia pastoris operably linked to
(b) a structural gene for HSA which includes the secretion signal sequence operably linked to
(c) a 3' termination sequence of AOX1 isolated from Pichia pastoris ligated to
(d) at least one marker gene,
(e) a second DNA fragment which is about 0.19 kilobase sequence of an autonomous replicating DNA sequence.

8. A linear integrative site-specific vector comprising the following serial arrangement:
(a) a first insertable DNA fragment,
(b) at least one expression cassette containing a structural gene for HSA which includes the secretion signal sequence, operably linked to a regulatory region and a 3' termination sequence, and
(c) a second insertable DNA fragment.

9. The vector of claim 8, wherein said vector is defined as in any of claims 4 to 6.

10. A plasmid comprising the following:
(a) an autonomously replicating sequence capable of replication in methylotrophic yeasts and
(b) at least one expression cassette containing a structural gene for HSA which includes the secretion signal sequence, operably linked to a regulatory region and a 3' termination sequence.

11. The plasmid of claim 10, wherein said expression cassette is defined as defined in claim 5.

12. Methylotrophic yeast of Pichia pastoris transformed with at least one vector containing at least one expression cassette comprising a regulatory region operably linked to a structural gene for HSA which includes the secretion signal sequence, operably linked to a 3' termination sequence.

13. The methylotrophic yeast of claim 12, wherein the yeast is Pichia pastoris strain GS115 (NRLL Y-15851).

14. The methylotrophic yeast of claim 12 or 13, wherein said vector is at least one linear integrative site-specific vector as defined in any of claims 3 to 6.

15. The yeast of claim 12 or 13, wherein said vector is at least one plasmid as defined in claim 10 or 11.
